# EUROPÄISCHE PATENTSCHRIFT

(11) **EP 0 707 655 B1**
(45) Veröffentlichungstag und Bekanntmachung des Hinweises auf die Patenterteilung: **28.06.2000**
(21) Anmeldenummer: 95915834.6
(22) Anmeldetag: 31.03.1995
(51) Int. Cl.: C12N 15/86, C12N 9/00, C07K 14/00, A61K 48/00

(54) **DNA-EXPRESSIONSVEKTOREN ZUR VERWENDUNG IN DER GENTHERAPEUTISCHEN BEHANDLUNG VON GEFÄSSERKRANKUNGEN**
DNA EXPRESSION VECTORS FOR USE IN THE TREATMENT OF VASCULAR DISEASES BY GENE THERAPY
VECTEURS D'EXPRESSION A ADN POUR L'UTILISATION DANS LE TRAITEMENT PAR THERAPIE GENIQUE DE MALADIES VASCULAIRES

(30) Priorität: 31.03.1994 DE 4411402
(43) Veröffentlichungstag der Anmeldung: 24.04.1996
(73) Patentinhaber: Schrader, Jürgen, Prof. Dr., 40597 Düsseldorf (DE)
(72) Erfinder: SCHRADER, Jürgen, D-40597 Düsseldorf (DE); GÖDECKE, Axel, D-40593 Düsseldorf (DE)
(74) Vertreter: Dost, Wolfgang, Dr.rer.nat., Dipl.-Chem.
(86) Internationale Anmeldenummer: EP9501202
(87) Internationale Veröffentlichungsnummer: WO9527070

(56) Entgegenhaltungen:
- WO-A-94/24269
- FEBS LETTERS, Bd. 316, Nr. 2, Januar 1993 Seiten 175-180, M. NAKANE ET AL. 'Cloned human brain nitric oxide synthase is highly expressed in skeletal muscle'
- PROC.NATL.ACAD.SCI.USA, Bd. 89, August 1992 Seiten 6711-6715, C.J. LOWENSTEIN ET AL. 'Cloned and expressed macrophage nitric oxide synthase contrasts with the brain enzyme'
- J.BIOL.CHEM., Bd. 267, Nr. 21, Juli 1992 Seiten 14519-14522, S.P. JANSSENS ET AL. 'Cloning and Expression of a cDNA Encoding Human Endothelium-derived Relaxing Factor/Nitric Oxide synthase'
- BIOCHEM.BIOPHYS.RES.COMMUN., Bd. 196, Nr. 3, November 1993 Seiten 1481-1489, I.G. CHARLES ET AL. 'Cloning and Expression of a Rat Neuronal Nitric Oxide Synthase Coding Sequence in a Baculovirus/Insect Cell System'
- JOURNAL OF THE AMERICAN COLLEGE OF CARIOLOGY, Bd. 1A, Nr. 484A, Februar 1994 Seite 235a L.J. FELDMAN ET AL. 'Site specificity of adenovirus-mediated gene transfer by hydrogel-coated balloon. A histochemical and PCR analysis. Abstract No: 906-34'
- CIRCULATION, Bd. 88, Nr. 4(2), Oktober 1993 Seite I-660 P.G. STEG ET AL. 'Local Delivery of Adenovirus for percutaneous Arterial Gene Transfer. A Comparison of Double and Hydrogel-Coated Balloons. Abstract No: 3554'
- ARCHIVES OF SURGERY, Bd. 128, November 1993 Seiten 1212-1220, D.A. GELLER ET AL. 'Should Surgeons Clone Genes?'
- DATABASE EMBL MAMMALIAN Accession no. M95674, 24.Juni 1992 & W.C. Sessa et al. 'Molecular cloning and expression of a cDNA encoding endothelial cell nitric oxide synthase' (Unpublished)

## Beschreibung

Die Erfindung betrifft die Verwendung von DNA-Expressionsvektoren zur gentherapeutischen Behandlung von Gefässerkrankungen, wie Bluthochdruck, Arteriosklerose, Stenose oder Restenose.

Unter der perkutanen transluminalen koronaren Angioplastie (PTCA) versteht man ein in der Klinik weit verbreitetes Verfahren bei dem die kritische Einengung eines Herzkranzgefässes (Koronarstenose) über einen Katheter mechanisch aufgedehnt und damit wieder durchgängig gemacht wird. Die Achillesferse der PTCA ist bis zum heutigen Tag die Restenose. Diese tritt nach Wochen bis Monaten nach erfolgreicher PTCA in 12-43% aller behandelten Patienten auf (Lange R.A.et al, Southwestern Internal Medicine Conference: Restenosis: The achilles heal of coronary angioplasty, Am.J. Med. Sci 265-274, 1993). Eine erneute PTCA oder eine Bypass-Operation ist die notwendige Folge.

Der genaue Mechanismus der Restenose ist letztlich ungeklärt. Entscheidend ist aber der initiale mechanische Dehnungsreiz bei dem es neben der Zerstörung der Endothelzellen zu Rissen in den arteriosklerotischen Wandbezirken bei gleichzeitiger Ablagerung von Fibrin und Blutplättchen kommt. Die Blutplättchen setzen ihrerseits gefässkonstriktorische und die Proliferation steigernde Stoffwechselprodukte frei, die die wachstumshemmenden Faktoren der glatten Gefässmuskulatur überspielen. Eine Hyperplasie der Muskelmedia, d.h. eine Restenose ist die Folge.

Verschiedene Strategien zur Verhinderung der Restenose wurden in der Vergangenheit in klinischen Studien untersucht. Was die pharmakologische Therapie betrifft, so hatte weder der Einsatz von antithrombotischen (Aspirin, Heparin), antispastischen (Nifedipin, Diltazem), antiproliferativen (Colchizin) Mitteln noch eine Lipidspiegel sendende Therapie einen günstigen Einfluss auf die Entwicklung der Restenose. Ebenso erbrachte die Entwicklung neuer Katheter (Laser-Angioplastie, Atherektomie Katheter) zur Minimalisierung des Gewebetraumas bei der PTCA keinen Erfolg bei der Verhinderung der Restenose.

Eine Therapie die Restenose gibt es z.Z. nicht. Dies liegt wahrscheinlich daran, dass die Wirkspiegel im Blut nach systemischer Applikation eines Pharmakons zu gering sind, um lokal therapeutisch wirksam werden zu können.

Stickstoffmonoxid (NO) wird in Säugetierzellen aus der Aminosäure L-Arginin unter Vermittlung des Enzyms NO-Synthase (NOS) gebildet. NO ist ein wichtiger Botenstoff bzw. Signalmolekül im menschlichen Körper, das eine Vielzahl physiologischer und pathophysiologischer Wirkungen vermittelt. Im zentralen Nervensystem ist NO wahrscheinlich an der Regulation von integrativen Leistungen, d.h. Gedächtnisfunktionen beteiligt. Im Gastrointestinaltrakt vermutet man eine Beteiligung an der Darmperistaltik. Das von Makrophagen gebildete NO ist in der Lage, Bakterien und Parasiten abzutöten. Innerhalb des Herz-Kreislaufsystems wird NO von den Endothelzellen gebildet, wo es mindestens zwei wichtige Funktionen ausübt. Zur luminalen Seite des Gefässes hin hemmt es die Plättchenaggregation und ist damit für die antithrombogene Eigenschaft der Gefässinnenwand mitverantwortlich. An der abluminalen Seite relaxiert NO die glatte Gefässmuskulatur und übt langfristig einen proliferationshemmenden Effekt aus. Ein Wegfall des endothelialen NO, z.B. bei Schädigung des Blutgefässendothels, führt am Gesamtorganismus zu einem Bluthochdruck und ist wahrscheinlich auch an der Entstehung der Arteriosklerose beteiligt. Für die Funktion von NO ist ausserdem wichtig, dass seine biologische Halbwertzeit kürzer als eine Sekunde ist. Somit kann NO lediglich die Zellen in unmittelbarer Nachbarschaft vom Bildungsort erreichen, d.h. die Wirkung von NO ist lokal beschränkt.

Zur Familie der NOS gehören mindestens drei verschiedene NOS Isoenzyme: das endotheliale Enzym (eNOS), das Gehirn-Enzym (bNOS) und die induzierbare NOS (iNOS). Alle drei Isoenzyme sind inzwischen isoliert, ihre Primärstruktur, d.h. Aminosäuresequenz aufgeklärt und die kodierenden Genabschnitte charakterisiert. Der wesentliche Unterschied zwischen diesen NOS liegt in ihrem Molekulargewicht und vor allem in der Regulation der Expression und der enzymatischen Aktivität. So werden die eNOS und bNOS über ihre Aktivität und die iNOS vorzugsweise über die Expression reguliert.

Zur Charakterisierung der NOS-Enzyme wird folgendes erläutert:
eNOS: Dieses Isoenzym hat ein Molekulargewicht von 133 kDa, hat eine Bindungstelle für Calmodulin, die abhängig ist von der freien Ca++-Konzentration und liegt zu mehr als 90% membrangebunden vor.
bNOS: das Hirn-Enzym ist ein Homodimer mit einem Molekulargewicht von 160 kDa pro Untereinheit, das zu weniger als 10% membrangebunden vorliegt. Wie bei der eNOS ist die Calmodulin-Bindung abhängig vom freien Ca++, d.h. beide Enzyme werden nur aktiv, wenn die intrazelluläre Ca++-Konzentration, z.B. infolge eines rezeptorvermittelten Ca++-Einstroms, erhöht ist.
iNOS: die induzierbare NOS ist ebenfalls ein Homodimer mit einem Molekulargewicht von 130 kDa pro Untereinheit. Der wesentliche Unterschied zu den anderen Isoenzymen ist, dass die Aktivität der iNOS unabhängig von Calmodulin und damit unabhängig vom zellulären Calcium ist. Weil die Umsatzgeschwindigkeit für L-Arginin für die iNOS etwa 10-100 mal höher ist als bei der eNOS oder bNOS, wird die iNOS auch als "high output" NOS bezeichnet. Die iNOS lässt sich unter basalen Bedingungen normalerweise nicht nachweisen; sie wird jedoch nach immunologischer Aktivierung durch Entzündungsmediatoren und Endotoxine stark exprimiert.

Aufgabe der Erfindung ist es, einen Expressionsvektor zur Verfügung zu stellen, welcher geeignet ist, zur gentherapeutischen Behandlung von Gefässerkrankungen, insbesondere Bluthochdruck oder Arteriosklerose, Stenose oder Restenose.

Die vorstehende Aufgabe wird gelöst durch einen DNA-Expressionsvektor, der aus einer DNA-Sequenz, welche für ein Protein codiert, das die biologische Aktivität von Stickstoff-monoxid-synthase beinhaltet, und aus eukaryotischen Regulations-Elementen besteht, wobei die mit diesem DNA-Expressionsvektor transformierten oder infizierten Blutgefässe rekombinante Stickstoff-monoxid-synthase in einer therapeutisch annehmbaren Menge zur Hemmung von Stenose oder Restenose der genannten Blutgefässe und/oder zur Hemmung der Proliferation der glatten Gefässmuskulatur exprimieren.

Der vorstehende Expressionsvektor findet gemäss der Erfindung. Verwendung in einem Verfahren zur Behandlung von Gefässerkrankungen, insbesondere Bluthochdruck, Arteriosklerose oder Stenose, und insbesondere für Restenose der karonaren Herzkranzgefässe nach perkutaner, transluminaler koronarer Angioplastie.

Im weiteren wird eine pharmazeutischen Zusammensetzung zur Verfügung gestellt.

Die Erfinder haben überraschenderweise gefunden, dass die Expression von rekombinanter Stickstoff-monoxid-synthase in Blutgefässen, die mit einem hier beschriebenen DNA-Expressionsvektor transfiziert wurden, zu einer therapeutisch relevanten Hemmung der Gefäss-Stenose und Restenose nach perkutaner, transluminaler, koronarer Angioplastie führt.

Die Erfindung bezieht sich auf eine DNA-Sequenz, vorzugsweise eine cDNA-Sequenz, die erhalten wird von Säugern, vorzugsweise von Mäusen oder Menschen, welche für iNOS-, bNOS- oder eNOS-, vorzugsweise iNOS-Aktivität codiert. Bevorzugt wird die iNOS cDNA eingesetzt, da sie eine höhere spezifische Aktivität hat, die darüber hinaus Ca-unabhängig ist. Die Enzymaktivität ist daher unabhängig von regulatorischen Einflüssen.

Der DNA-Expressionsvektor kann ein Sequenzelement, das die Replikation in Bakterien ermöglicht, ein Sequenzelement, das die Replikation des genannten Vektors in eukaryotischen Zellen ermöglicht, vorzugsweise das SV40 Replikations-Sequenzelement, ein Polyadenylierungs-Signal und ein oder mehrere Introns umfassen.

Das Regulationselement kann den Promotor und/oder die Enhancer-Region von eukaryotischen Viren, vorzugsweise von Cytomegalovirus oder Adenovirus, und mehr bevorzugt den Promotor-Enhancer des Cytomegalovirus Immediate-Eearly Polypetid-Gens, umfassen.

Ausserdem bezieht sich die vorliegende Erfindung auf die Behandlung und Verhinderung von Gefässerkrankungen beim Menschen, wie Bluthochdruck und Arteriosklerose sowie Stenose oder Restenose der Blutgefässe. Insbesondere betrifft die Erfindung ein Verfahren zur Behandlung und Vorbeugung von Restenose der Herzgefässe nach perkutaner, transluminaler koronarer Angioplastie (PTCA), wobei die Blutgefässe mit dem oben beschriebenen DNA-Expressionsvektor in Kontakt gebracht, tranfiziert oder infiziert werden .

Die Transfizierung oder Infizierung der Blutgefässe mit einem DNA-Expressionsvektor kann durch jede im Stand der Technik bekannte Standardmethode erfolgen und ist nicht auf die hier beschriebene Transfizierungstechnik beschränkt.

Weiterhin betrifft die Erfindung eine pharmazeutische Zusammensetzung, die diesen DNA-Expressionsvektor zur Behandlung oder Verhinderung der oben genannten Gefässerkrankungen umfasst. Die pharmazeutische Zusammensetzung kann zusätzlich zu dem DNA-Expressionsvektor einen pharmazeutisch annehmbaren Träger, ein Stabilisierungsmittel oder Puffer umfassen.

Die nachfolgenden Figuren dienen der Erläuterung der Erfindung.

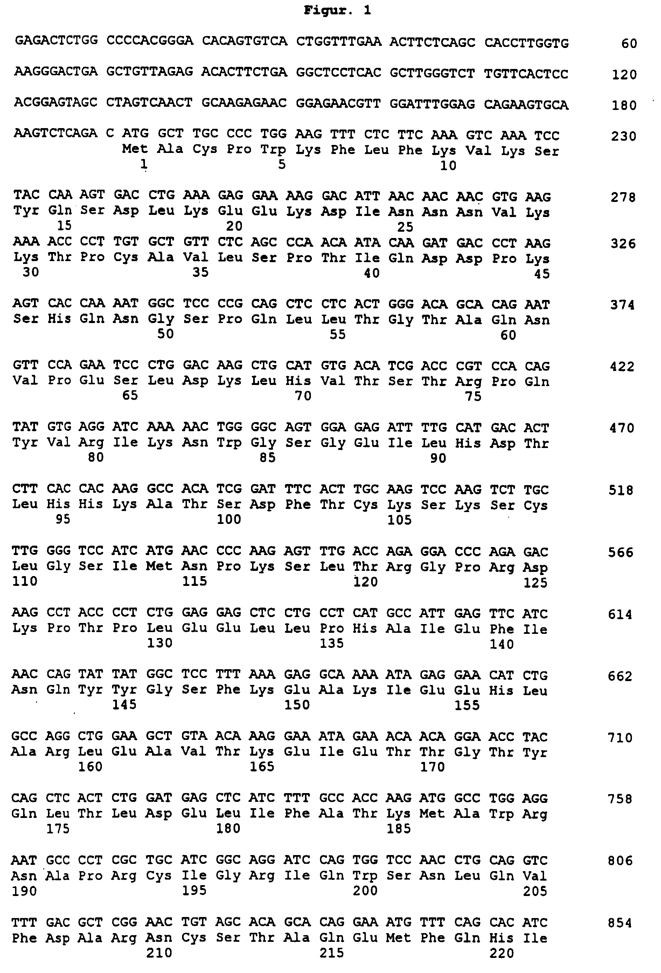

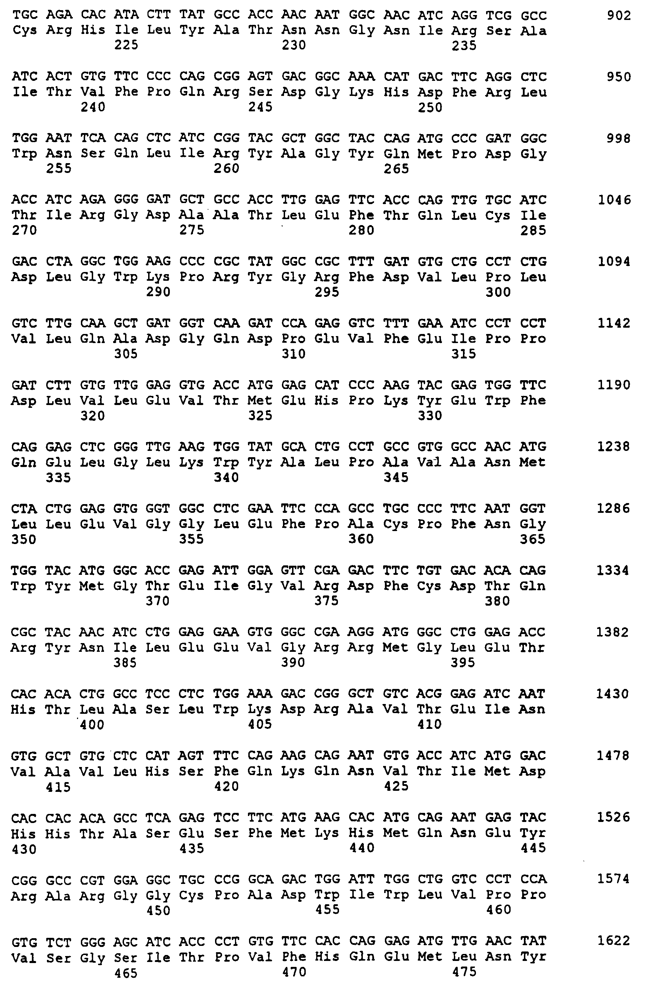

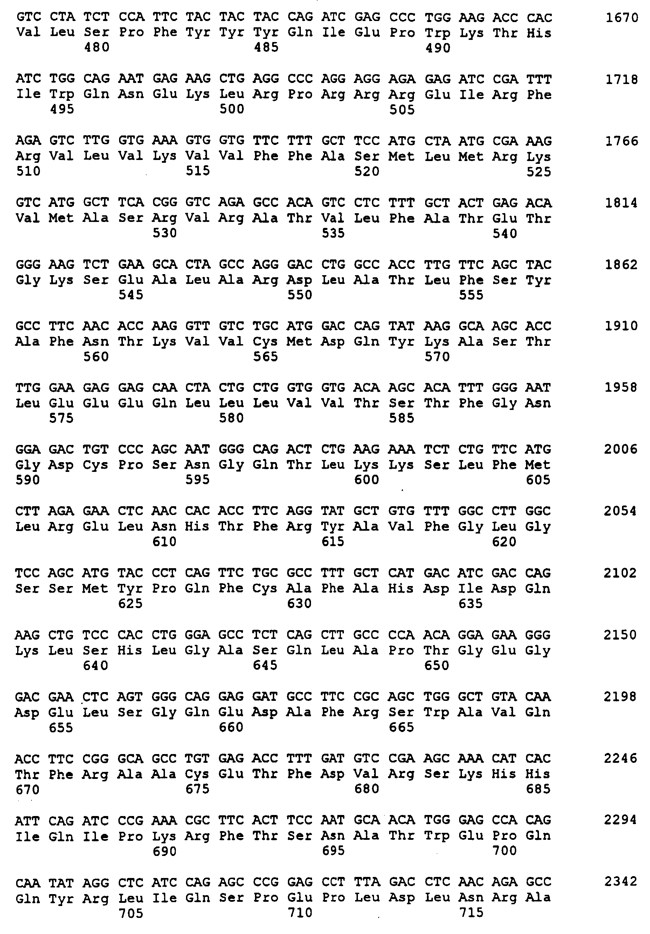

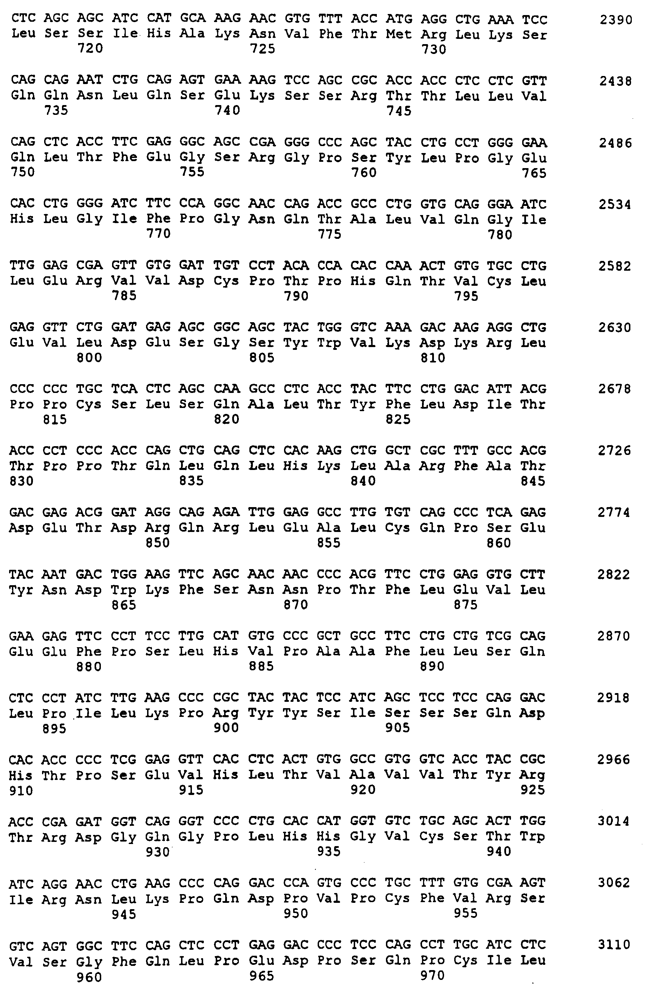

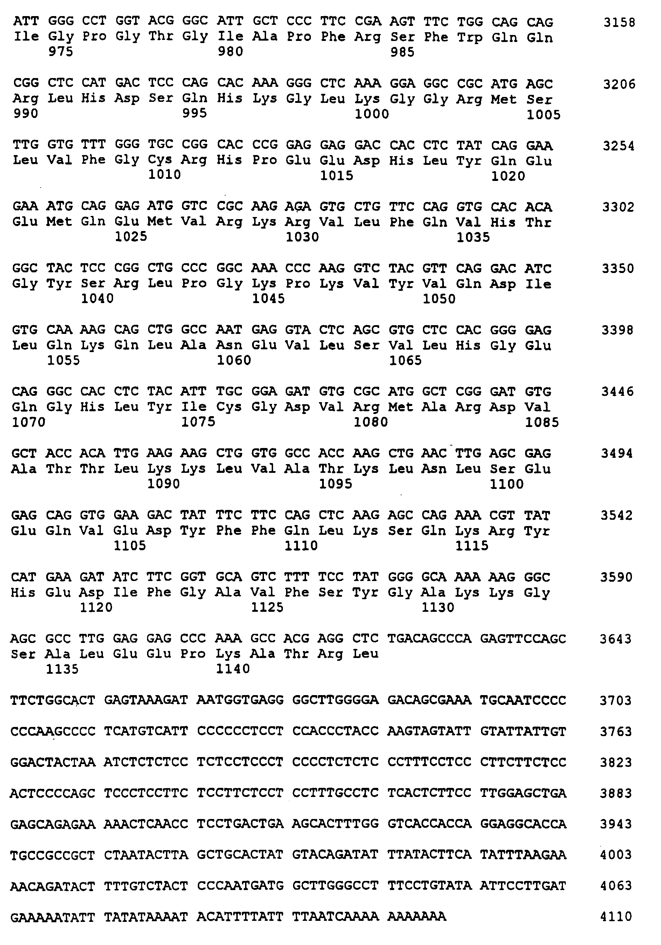

- Fig.1: Nukleotid- und Aminosäuresequenz der induzierbaren NO-Synthase der Maus (Mus musculus/Mammalia) abgeleitet aus der clonierten cDNA, Genbank Accession No.: M92649);

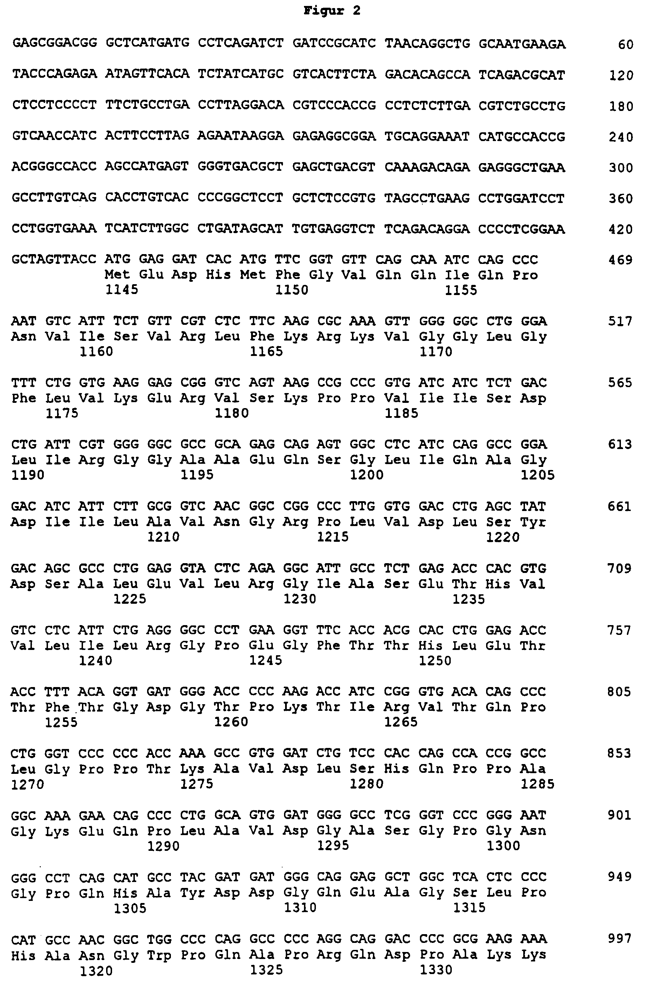

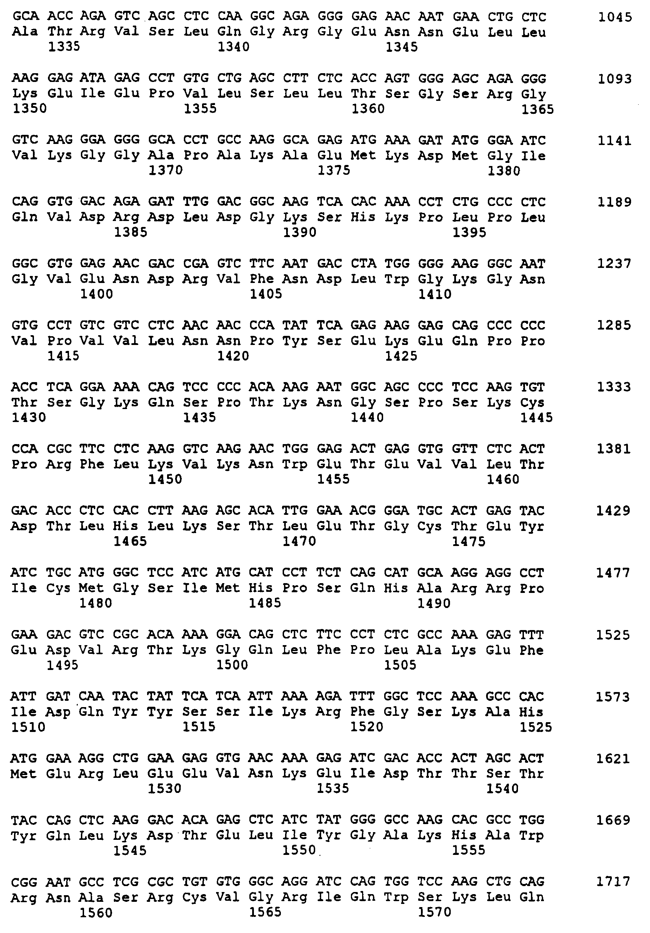

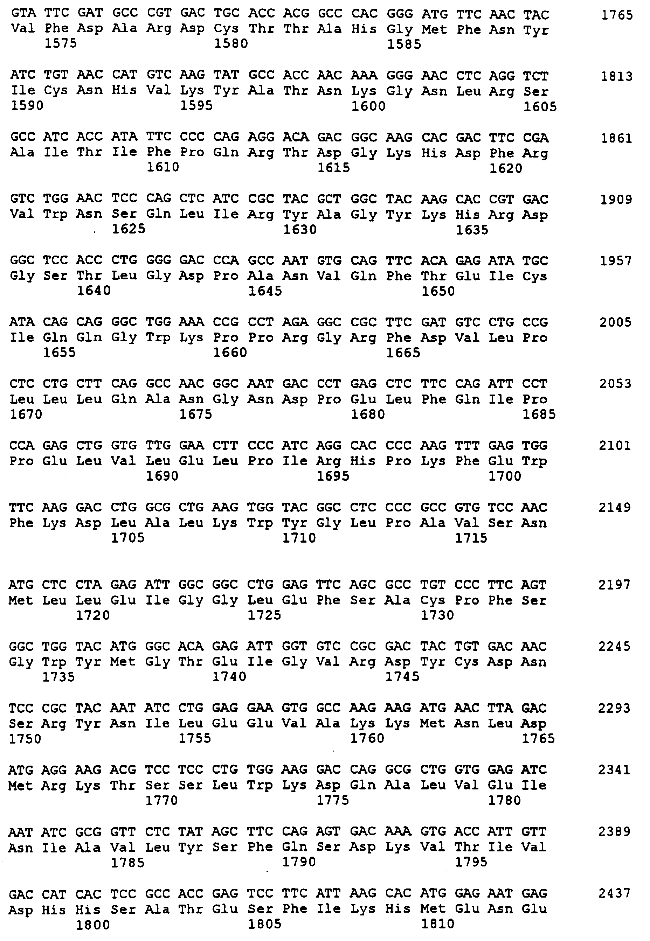

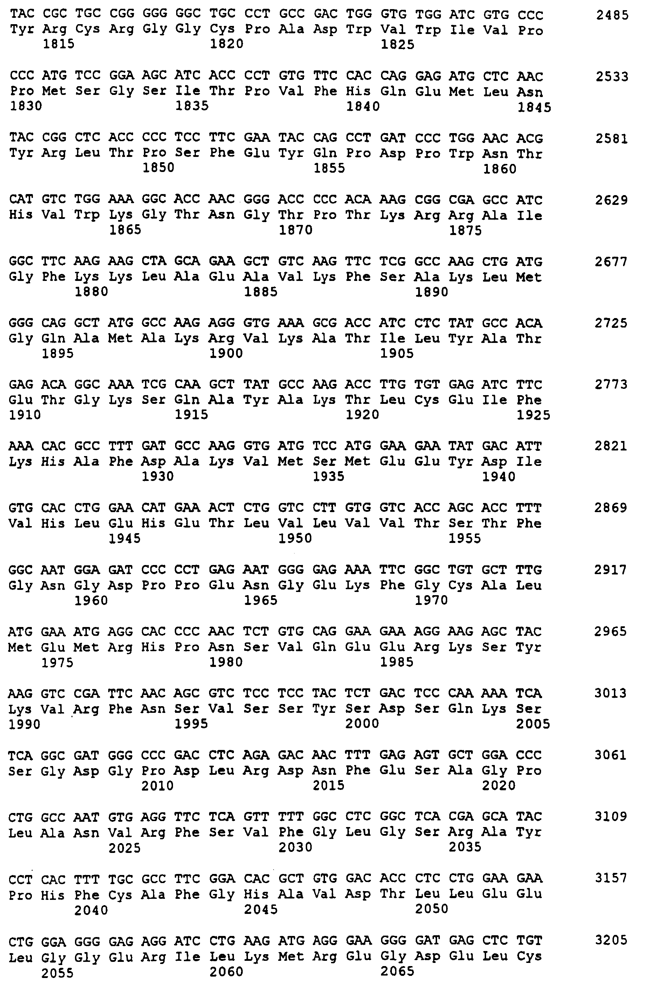

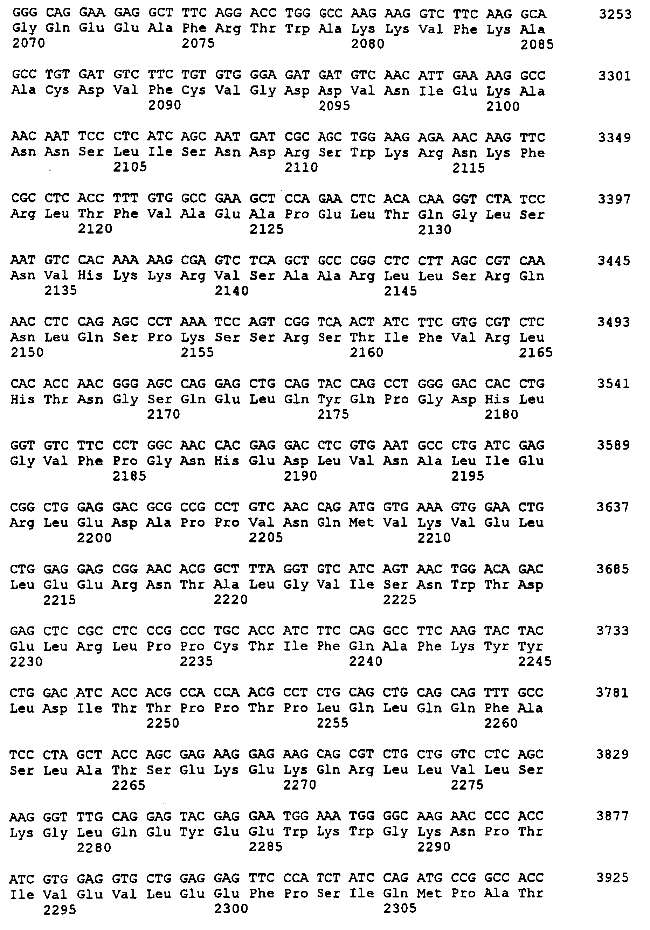

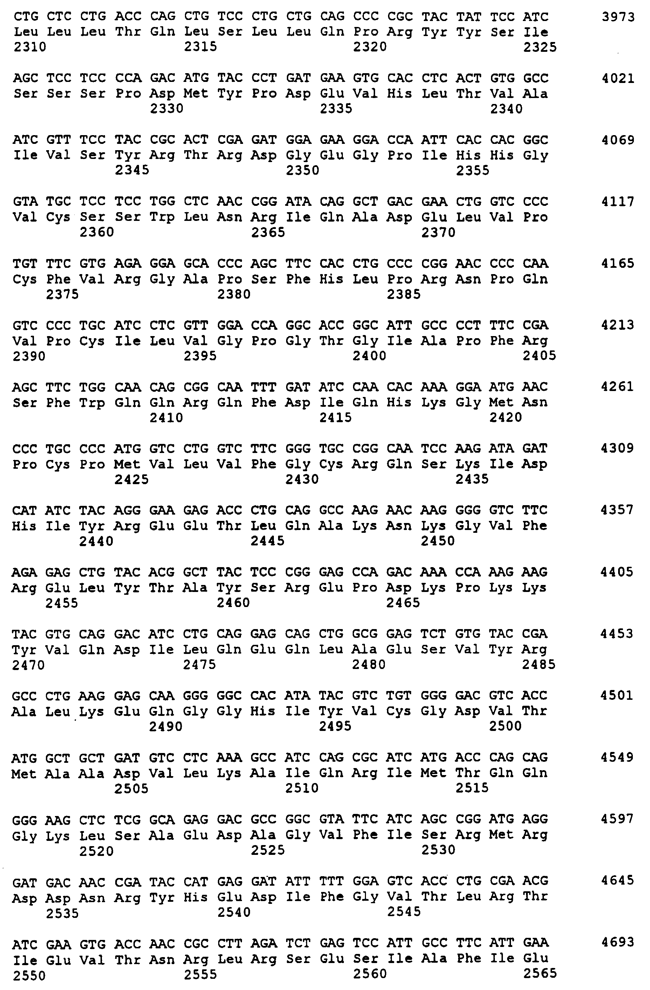

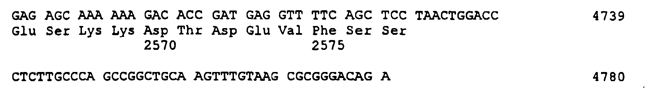

- Fig.2: Nukleotid- und Aminosäuresequenz der neuronalen NO-Synthase des Menschen (Homo sapiens/Mammalia) aus der clonierten cDNA, Genbank Accession No.:L02881);

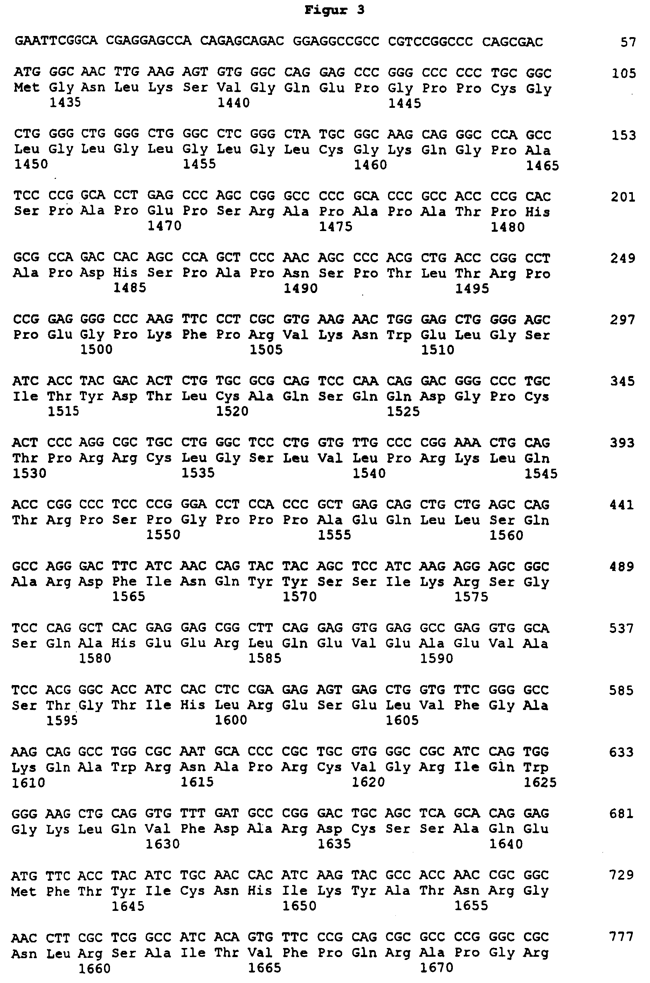

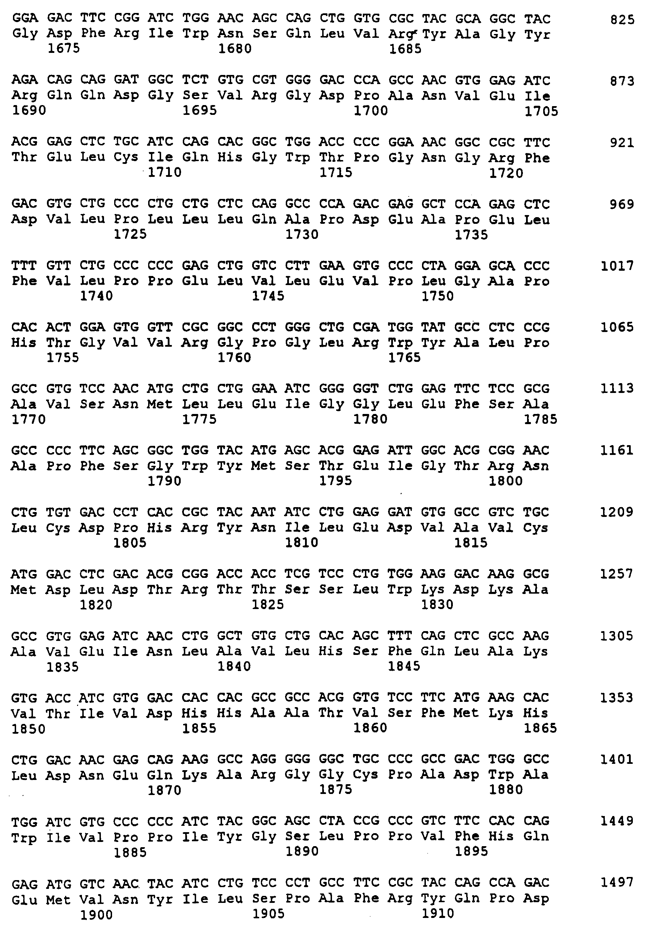

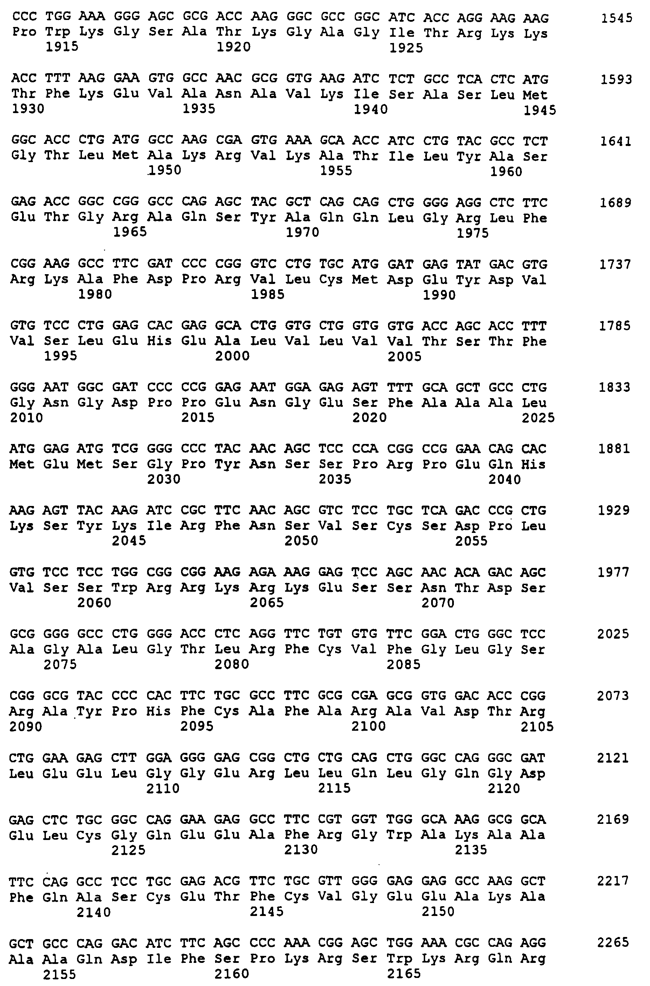

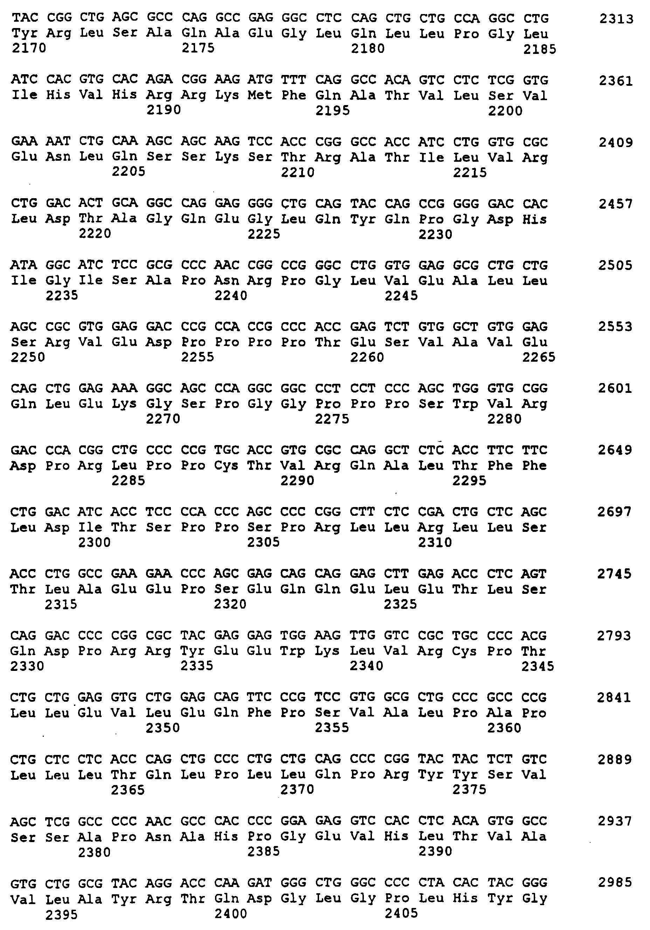

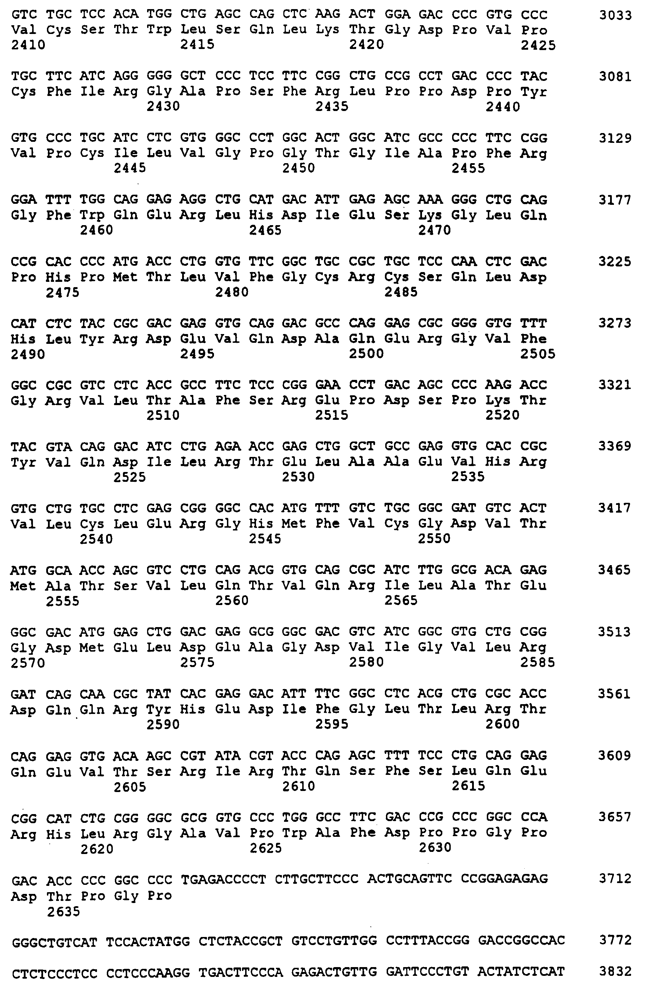

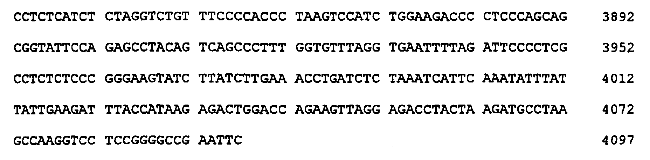

- Fig.3: Nukleotid- und Aminosäuresequenz der endothelialen NO-Synthase des Rindes (Bos taurus/Mammalia) abgeleitet aus der clonierten cDNA, Genbank Accession No.: M95674;
- Fig.4a: das 2,17 Kb CMV-Promotor enthaltende PvuI-PvuII-Fragment, das zum weiteren Aufbau des Plasmids pSCMV aus dem Plasmid pSP65h-CMV isoliert wurde;
- Fig.4b: das die 2,80 Kb 3'-Region des β-Globin-Gens und den SV40 Replikationsursprung enthaltende PvuI-PvuII-Fragment, das zum weiteren Aufbau des Plasmids pCMV aus dem Plasmid pSCT GAL X-556 isoliert wurde;
- Fig.4c: das Plasmid pSCMV; Fig.4d das 3,97Kb iNOS-enthaltende HindIII-XhoI-Fragment gemäss Fig. 1;
- Fig.4e: das Plasmid pSCMV-iNOS.

Die nachfolgenden Beispiele sollen die Erfindung näher erläutern, ohne sie jedoch darauf zu beschränken.

### Beispiel 1

### I. Elemente eines Expressionsvektors

Das Expressionsplasmid pSCMV-iNOS wurde auf der Basis des Plasmids pSO65 (Promega Biotech) cloniert. Es enthält die folgenden funktionellen Elemente:
- Promotor/Enhancer des humanen Cytomegalovirus Immediate-Early-Polypeptids (Pos. 216-809 /Genbank Accession No.: K03104)
- cDNA der induzierbaren NO-Synthase der Maus (Pos. 127-4110/Genbank Accession No.: M92649)
- genomische Sequenzen des ss-Globin-Gens des Kaninchens (Pos. 905-2080), die das Intron 2, das Exon 3 und das Polyadenylierungssignal enthalten (Pos. 905-1827 sind unter Genbank Accession No.: J00659 abgespeichert).
- den Replikationsursprung des SV40-Virus (Pos. 130 [SV40 early map] bzw. Pos. 5176- 130 der zirkulären SV40 Karte/Genbank Accession No.: V01380).

### II. Herstellung eines Expressionsvektors:

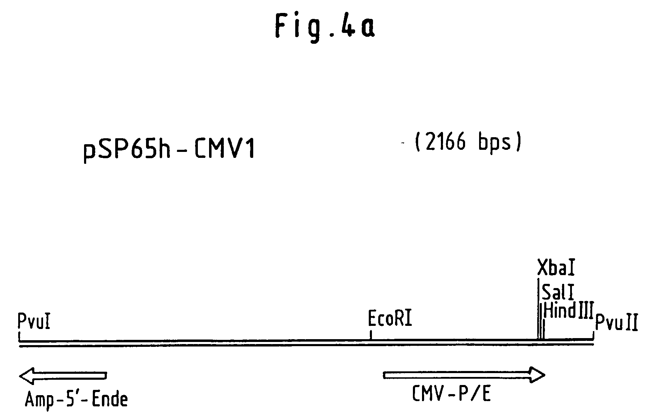

Der CMV-Promotor/Enhancer lag als DNA-Fragment mit stumpfen Enden in die Smal-Stelle des Plasmids pSP65 inseriert vor (pSP65 h-CMV1) Aus diesem Plasmid wurde das PvuI-PvuII-Fragment isoliert (Fig.4a), das den CMV-Promotor trägt.

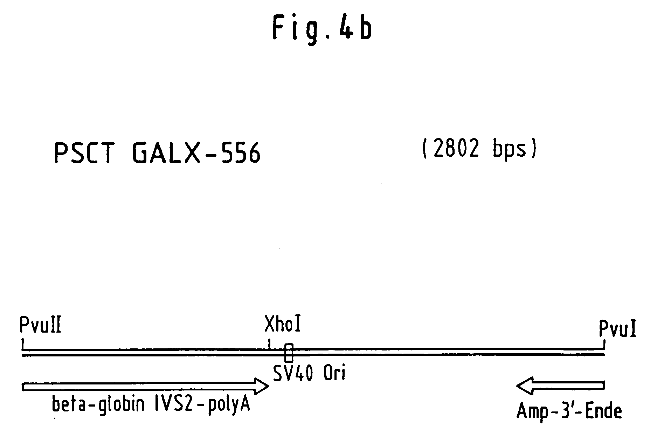

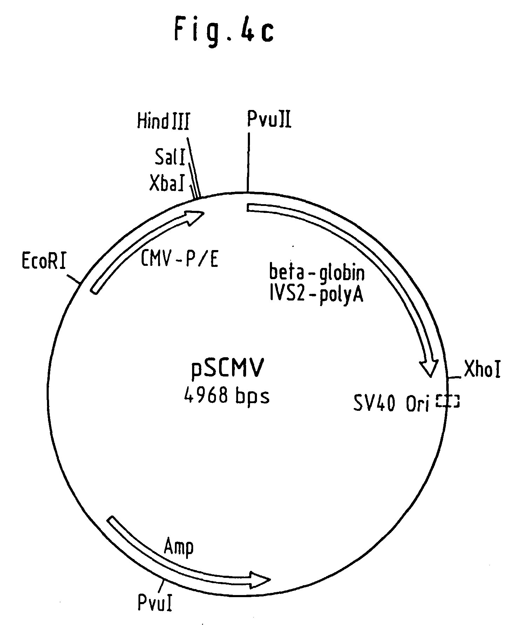

Aus dem Plasmid PSCT GAL.X-556 (siehe Fig. 4b) wurde ein PvuII-PvuI-Fragment isoliert, das die 3'-Region des ss-Globin-Geris und den SV40 Replikationsursprung trägt (Fig.4b). Dieses Fragment wurde mit dem PvuII-PvuI-Fragment aus pSP65h-CMV1 ligiert. An dem resultierenden Plasmid (pSCMV, Fig. 4c) wurden folgende Veränderungen vorgenommen:
- Deletion der Vektorsequenzen zwischen HindIII und PvuII bei gleichzeitiger Insertion eines ClaI-Linkers. Dadurch gehen die Ausgangsschnittstellen PvuII und HindIII verloren.
- Umwandlung der Xhol-Schnittstelle in eine PvuI-Schnittstelle.

Der erhaltene Vektor wurde als pSCMV2 bezeichnet.

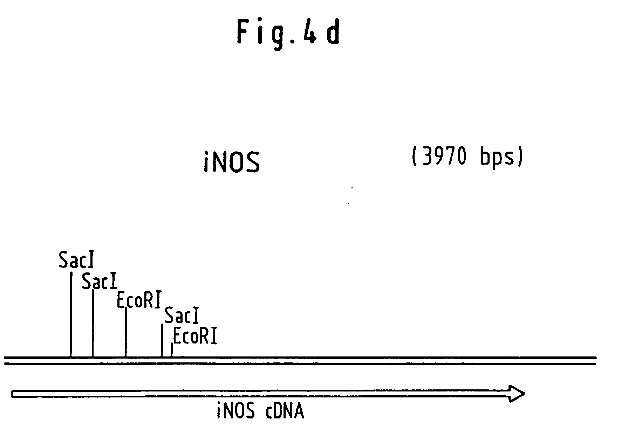

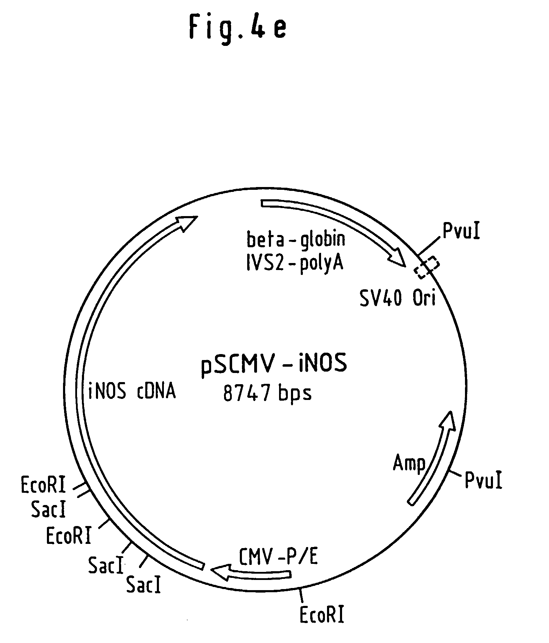

Die iNOS-cDNA wurde als HindII-Xhol-Fragment (Fig.4d) in den XbaI-SaII verdauten Vektor pSCMV2 cloniert. Dazu wurden die 5'-überhängenden Enden nach der XbaI-Verdauung durch Auffüllen in glatte Enden umgewandelt. Am 3'-Ende wurde die cDNA mit dem Intron und dem Polyadenylierungssignal des ss-Globin-Gens aus dem Huhn fusioniert. Die Funktionalität des Konstrukts wurde durch Transfektion von COS-Zellen und anschliessendem Nachweis der Enzymaktivität überprüft. Das resultierende Plasmid pSCMV-iNOS (Fig.4e) wurde für die Transfektionsexperimente eingesetzt. Der CMV-Promotor vermittelt eine konstitutive Expression des iNOs-Gens. Die Aktivität des gebildeten Enzyms ist Ca-unabhängig.

### III. Herstellung von DNA-Liposomenkomplexen

Die Anzucht von Sendai-Viren erfolgte in der Chorionallantis-Flüssigkeit befruchteter Hühnereier (d10-d12 der Embryonalentwicklung) nach Standardverfahren (Nakanishi M., Uchida T., Sugawa H., Ishiura M., Okada Y., Exp. Cell. Res. 159, 399-409).

Phosphatidylcholin, Phosphatidylserin und Cholesterin wurden in Tetrahydrofuran gelöst, im molaren Verhältnis von 4.8:1:2 gemischt und unter Stickstoff evaporiert. 10 mg der getrockneten Lipide wurden durch Vortexen in 200 µl pSCMV-iNOS [gelöst in BSS (140 mM NaCl, 5.4 mM KCl und 10 mM Tris-HCl, pH7.69)] (Konz. 1 µg/µl) suspendiert. Die erhaltenen Liposomen wurden mit 4 ml Sendai-Virus (Z-Stamm) (16000 Hämagglutinations-Einheiten (HAU)/ml) 3h bei 37°C unter leichtem Schütteln inkubiert und anschliessend für 20'' im Ultraschallbad beschallt. Die Viren wurden vor der Verwendung mit UV-Licht inaktiviert (11 J/m² x s). Die erhaltene Suspension wurde auf zwei Saccharose-Gradienten (1 ml 60%, 1 ml 40%, 8 ml 30% Saccharose in BSS) geladen und für 3 h bei 30.000 UpM im SW40-Rotor (Beckman Instruments) bei 4°C zentrifugiert. Die oberen 2 ml wurden abgenommen und für die Transfektionsexperimente eingesetzt.

### IV. Transtektions-Protokoll

Zur Untersuchung des Effekts einer lokalen Überexpression der iNOS auf die Proliferation der glatten Gefässmuskulatur nach Endothelschädigung in vivo wurde das von Clowes AW, Reidy MA und Clowes (MM Lab. Invest 49, 327-333, 1983) etablierte Restenose-Modell der Ratte benutzt. Im Bereich des Halses wurde die Arteria carotis communis dargestellt sowie die rostral gelegene Bifurkation, an der sich die A.carotis communis in die A. carotis externa und A. carotis internA aufspaltet. Die A. carotis externa wurde etwa 1 cm rostral von der Bifurkation permanent, die A. carotis interna durch eine Gefässklemme vorübergehend unterbunden. Die A. carotis externa wurde durch einen Schnitt etwa 0.5 cm rostral von der Bifurkation geöffnet. Über diese Öffnung wurde ein Fogarty 2F Embolektomie-Katheter in die A. carotis communis eingeführt, und das Endothel der A. carotis communis wurde durch drei Passagen des dilatierten Katheters entfernt.

Für die "Therapiegruppe" wurde das folgende Verfahren angewendet. Das denudierte Gefäss wurde nach Entfernen des Katheters 1.5 bis 2 cm caudal von der Bifurkation mit einer Gefässklemme verschlossen. Von der Öffnung in der A. carotis externa wurde ein Polyethylen-Katheter in das Gefäss eingebunden. Über diesen Katheter wurden 50-100 µl einer Lösung mit DNA-Liposomen-Komplexen innerhalb 15-20 min. in den isolierten Abschnitt der A. carotis communis injiziert. Es wurde darauf geachtet, dass der Gefässabschnitt während der Transfektion durch die Liposomenlösung unter Druck stand. Nach Entfernen des Transfektionkatheters wurde die A. carotis externa caudal des Gefässschnittes permanent unterbunden und anschliessend der Blutfluss durch Entfernen der Gefässklemmen über die A. carotis communis und A. carotis interna wieder hergestellt.

### V. Morphologische Analyse der Gefässe

Ratten wurden zu unterschiedlichen Zeitpunkten (bis 21d) nach Transfektion der A. carotis communis auf die morphologischen Veränderungen in den Gefässen hin untersucht. Die Gefässe wurden durch Perfusion mit 3% Paraformaldehyd in PBS (140 mM NaCl, 10 mM Na-Phosphat, pH 7.5) fixiert und geschnitten. Das Ausmass der Proliferation wurde mit kontrollbehandelten Gefässen verglichen. Es zeigte sich eine Reduktion der Stenose-Bildung um bis zu 60% bei den behandelten Tieren. Diese Studie zeigt erstmals, dass in diesem Tiermodell die lokale Überexpression eines Gens eine therapierelevante Hemmung der Gefässstenose zur Folge hat und demonstriert die besondere Rolle von NO für die Hemmung der Proliferation der glatten Gefässmuskulatur.

## Patentansprüche

1. Verwendung eines DNA-Expressionsvektors umfassend eine DNA-Sequenz, welche für ein Protein codiert, das die biologische Aktivität von Stickstoffmonoxidsynthase (NOS) besitzt, und eukaryotische Regulations-Elemente, wobei die genannten eukaryotischen Regulations-Expressions-Elemente die Expression der genannten DNA-Sequenz in eukaryotischen Zellen bewirken, bzw. eines pharmazeutischen Mittels, welches einen oben genannten DNA-Expressionsvektor und einen pharmazeutisch annehmbaren Träger umfasst, zur Herstellung eines Arzneimittels zur Behandlung oder Verhinderung von Gefässerkrankungen, Bluthochdruck oder Arteriosklerose, Stenose oder Restenose der Blutgefässe.

2. Verwendung nach Anspruch 1, wobei es sich bei der Restenose um eine Restenose der Herzkranzgefässe nach perkutaner transluminaler Koronar-Angioplastie (PTCA) handelt.

3. Verwendung nach Anspruch 1 oder 2, dadurch gekennzeichnet, dass der DNA-Expressionsvektor eine cDNA-Sequenz, welche für ein Protein codiert, das die biologische Aktivität von Stickstoff-monoxidsynthase (NOS) besitzt, und eukaryotische Regulations-Elemente umfasst, wobei die genannten eukaryotischen Regulations-Expressions-Elemente die Expression der genannten cDNA-Sequenz in eukaryotischen Zellen bewirken.

4. Verwendung nach einem der Ansprüche 1 - 3, wobei die genannte DNA- oder cDNA-Sequenz von Säugern abgeleitet ist.

5. Verwendung nach Anspruch 4, wobei die DNA- oder cDNA-Sequenz eine humane DNA- oder cDNA-Sequenz darstellt.

6. Verwendung nach einem Ansprüche 1 bis 4, wobei die cDNA die Nukleotidsequenz von Stickstoff-monoxid-synthase gemäss Fig. 1, Fig. 2 oder Fig. 3 umfasst, oder die Aminosäuresequenz von Stickstoff-monoxid-synthase gemäss Fig. 1, Fig. 2 oder Fig. 3 codiert.

7. Verwendung nach einem oder mehreren der vorangehenden Ansprüche, wobei die genannten eukaryotischen Regulations-Elemente von einem eukaryotischen Virus abgeleitet sind.

8. Verwendung nach einem oder mehreren der vorangehenden Ansprüche, wobei die genannten eukaryotischen Regulations-Elemente von dem Cytomegalovirus (CMV)-Promotor und/oder Enhancer der frühen Gene abgeleitet ist.

9. Verwendung nach Anspruch 7, wobei der genannte DNA-Expressionsvektor pSCMV-iNOS gemäß Fig. 4e darstellt.

10. Verwendung nach einem oder mehreren der Ansprüche 1 bis 7, wobei die genannten eukaryotischen Regulations-Elemente von einem Adenovirus-Promotor und/oder -Enhancer-Element abgeleitet sind.

## Claims

1. The use of a DNA expression vector comprising a DNA sequence which codes for a protein having the biological activity of nitrogen monoxide synthase (NOS), and eukaryotic regulation elements, the afore-mentioned eukaryotic regulation expression elements effecting the expression of said DNA sequence in eukaryotic cells, or of a pharmaceutical agent which comprises an above-named DNA expression vector and a pharmaceutically acceptable carrier, for the production of a medicament for treating or preventing vascular diseases, hypertension or arteriosclerosis, stenosis or restenosis of the blood vessels.

2. The use according to Claim 1, wherein the restenosis is a restenosis of the coronary vessels after percutaneous transluminal coronary angioplasty (PTCA).

3. The use according to Claim 1 or 2, characterised in that the DNA expression vector comprises a cDNA sequence which codes for a protein having the biological activity of nitrogen monoxide synthase (NOS) and eukaryotic regulation elements, with said eukaryotic regulation expression elements effecting the expression of said cDNA sequence in eukaryotic cells.

4. The use according to one of Claims 1 to 3, wherein said DNA or cDNA sequence is derived from mammals.

5. The use according to Claim 4, wherein the DNA or cDNA sequence represents a human DNA or cDNA sequence.

6. The use according to one of Claims 1 to 4, wherein the cDNA comprises the nucleotide sequence of nitrogen monoxide synthase according to Fig. 1, Fig. 2 or Fig. 3, or encodes the amino acid sequence of nitrogen monoxide synthase according to Fig. 1, Fig. 2 or Fig. 3.

7. The use according to one or more of the preceding claims, wherein said eukaryotic regulation elements are derived from a eukaryotic virus.

8. The use according to one or more of the preceding claims, wherein said eukaryotic regulation elements are derived from the cytomegalovirus (CMV) promoter and/or enhancer of the early gene.

9. The use according to Claim 7, wherein said DNA expression vector represents pSCMV-iNOS according to Fig. 4e.

10. The use according to one or more of Claims 1 to 7, wherein said eukaryotic regulation elements are derived from an adenovirus promoter and/or enhancer element.

## Revendications

1. Utilisation d'un vecteur d'expression à ADN comportant une séquence d'ADN codant pour une protéine, qui possède l'activité biologique de la (monoxyde d'azote)-synthase (NOS), et des éléments de régulation eucaryotes, lesdits éléments d'expression de régulation eucaryotes provoquant l'expression de la séquence d'ADN mentionnée ci-dessus dans des cellules eucaryotes, ou d'un agent pharmaceutique qui contient un vecteur d'expression à ADN mentionné ci-dessus et un support acceptable d'un point de vue pharmaceutique, pour préparer un médicament destiné au traitement ou à la prévention des maladies vasculaires, de l'hypertension ou de l'artériosclérose, de la sténose ou de la resténose des vaisseaux sanguins.

2. Utilisation selon la revendication 1, pour laquelle, pour ce qui concerne la resténose, il s'agit d'une resténose des artères coronaires après une angioplasie coronarienne transluminale percutanée (PTCA).

3. Utilisation selon la revendication 1 ou 2, caractérisée en ce que le vecteur d'expression à ADN comporte une séquence d'ADNc qui code pour une protéine ayant l'activité biologique de la (monoxyde d'azote)-synthase (NOS), ainsi que des éléments de régulation eucaryotes, lesdits éléments d'expression de régulation eucaryotes provoquant l'expression de la séquence d'ADNc mentionnée ci-dessus dans des cellules eucaryotes.

4. Utilisation selon l'une des revendications 1 à 3, pour laquelle ladite séquence d'ADN ou d'ADNc dérive de mammifères.

5. Utilisation selon la revendication 4, pour laquelle la séquence d'ADN ou d'ADNc représente une séquence d'ADN ou d'ADNc humaine.

6. Utilisation selon l'une des revendications 1 à 4, pour laquelle l'ADNc présente la séquence nucléotidique de la (monoxyde d'azote)-synthase seleon les Figures 1, 2 ou 3, ou encore code pour la séquence d'acides aminés de la (monoxyde d'azote)-synthase selon les Figures 1, 2 ou 3.

7. Utilisation selon l'une ou plusieurs des revendications précédentes, pour laquelle lesdits éléments de régulation eucaryotes dérivent d'un virus eucaryote.

8. Utilisation selon l'une ou plusieurs des revendications précédentes, pour laquelle lesdits éléments de régulation eucaryotes dérivent du promoteur de cytomégalovirus (CMV) et/ou de l'amplificateur des gènes précoces.

9. Utilisation selon la revendication 7, pour laquelle ledit vecteur d'expression à ADN représente le pSCMV-iNOS selon la Figure 4e.

10. Utilisation selon l'une ou plusieurs des revendications 1 à 7, pour laquelle lesdits éléments de régulation eucaryotes dérivent d'un élément promoteur et/ou amplificateur d'adénovirus.
